# EUROPEAN PATENT APPLICATION

(11) **EP 4 685 055 A1**
(43) Date of publication of application: **28.01.2026**
(21) Application number: 24190470.5
(22) Date of filing: 23.07.2024
(51) Int. Cl.: B64D 11/02, A61L 2/10, E03C 1/048

(54) **SINK UNIT FOR AN AIRCRAFT**

(71) Applicant: B/E Aerospace, Inc., Winston Salem, NC 27105 (US)
(72) Inventor: Dy, Sean Christopher, Lipa City, 4217 (PH); Don, Krisha Chrystelle, Tanauan, 4232 (PH); Pasia, Leandro, Rosario, 4225 (PH); Sto. Domingo, Richard, Taguig, 1633 (PH)
(74) Representative: Dehns

(57) **Abstract**

A sink unit (2) for an aircraft, wherein the sink unit (2) comprises:
a substantially horizontal countertop (4); and
a sink (6) extending downwards from the countertop (4);
wherein the sink (6) comprises an interior volume (8) defined by at least one side (10a-d) of the sink (6); and
wherein the sink (6) comprises:
a first aperture (12) configured to dispense water into the interior volume (8) of the sink (6);
a second aperture (14) configured to dispense heated air into the interior volume (8) of the sink (6); and
a UV light source (16a, 16b) configured to irradiate the interior volume (8) of the sink (6) with UV light;

wherein the first aperture (12), the second aperture (14) and the UV light source (16a, 16b) are located below the countertop (4) on the at least one side (10a-d) of the sink (6).

## Description

### Technical Field

This disclosure relates to a sink unit for an aircraft. In particular, this disclosure relates to a sink unit having apertures for water and heated air, and a UV light source located on a side of the sink.

### Background Art

The space available inside an aircraft is typically very limited. In particular, lavatory units on aircraft can be small spaces with a relatively large number of components inside. Therefore, some of the components of an aircraft lavatory may be more compact than those in a typical bathroom.

In particular, sinks on board aircrafts can be smaller than those in a typical bathroom. This can lead to water spilling or splashing over the side of the sink when a person uses the sink. This may lead to a perception that the aircraft lavatory is unclean. Furthermore, the presence of water on the floor could increase the risk of a person slipping or falling.

The present disclosure aims to address some of these problems by providing a sink unit for an aircraft.

### Summary of the Disclosure

According to the present disclosure, there is provided a sink unit for an aircraft, wherein the sink unit comprises:
a substantially horizontal countertop; and
a sink extending downwards from the countertop;
wherein the sink comprises an interior volume defined by at least one side of the sink; and
wherein the sink comprises:
   a first aperture configured to dispense water into the interior volume of the sink;
   a second aperture configured to dispense heated air into the interior volume of the sink; and
   a UV light source configured to irradiate the interior volume of the sink with UV light;
wherein the first aperture, the second aperture and the UV light source are located below the countertop on the at least one side of the sink.

The sink unit may be any suitable and desired type. In some examples, the sink unit may be configured for use by passengers on board an aircraft. For example, the sink unit may be part of a lavatory of an aircraft. The sink unit may be configured as a single (e.g. unitary, e.g. integrally moulded) piece or may comprise different parts which operate together as a sink unit.

The sink unit may be suitable for an aircraft owing to its size, shape, weight and/or the materials that it is formed from. For example, the size and shape of the sink unit may be adapted for the small space available in the lavatory of an aircraft. The sink unit may be formed from lightweight materials, as it is usually desirable to minimise the weight carried on board an aircraft. The materials may also be durable and easy to clean, such that they are suitable for frequent use and are intended to remain in use on board the aircraft for a long time (e.g. years).

The sink unit comprises a substantially horizontal countertop and a sink extending downwards from the countertop. The substantially horizontal countertop may be any suitable and desired type. In some examples, the countertop extends at least partially around the outermost perimeter of the sink (e.g. providing a lip around the sink). In some examples, the countertop may extend outwards from the sink and be configured to provide a surface to place objects (e.g. soap, handwash, tissues).

The countertop and the sink may be connected to one another in any suitable and desired way. In some examples, the countertop and the sink unit are formed as a unitary piece (e.g. a (e.g. integrally) moulded (plastic) part). In some examples, the countertop and the sink are separate pieces (e.g. being formed of different materials) and the countertop is shaped (e.g. comprising an aperture) to receive at least part of the sink.

The sink comprises at least one side. At least some of the sides of the sink may extend downwards from the substantially horizontal plane of the countertop. In some examples, at least some of the sides of the sink extend substantially vertically downwards from the countertop (e.g. such that the sides(s) of the sink meet the countertop at a right angle). However, it will be understood that the side(s) of the sink may have any suitable and desired shape. For example, the side(s) of the sink may be at least partially curved. In some examples, at least one of the sides of the sink may form (part of) the bottom of the sink.

The sink comprises an interior volume defined by at least one side of the sink. The interior volume of the sink may be defined by one or more sides of the sink and an (imaginary) surface in the plane of the countertop. The interior volume of the sink may be the volume of the space contained inside the sink, up to the plane of the countertop.

The sink comprises a first aperture configured to dispense water into the interior volume of the sink. The first aperture may be formed in the at least one side of the sink. The first aperture may be any shape and size. The first aperture may be in fluid communication with a source of water. The first aperture may form an outlet for water from a water source.

The sink comprises a second aperture configured to dispense heated air into the interior volume of the sink. The second aperture may be formed in the at least one side of the sink. The second aperture may be any shape and size. The second aperture may be in fluid communication with a source of heated air. The second aperture may form an outlet for heated air from a source.

The sink comprises a UV light source configured to irradiate the interior volume of the sink with UV light. The UV light source may be formed in the at least one side of the sink. In some examples, the UV light source may be at least partially recessed into the side of the sink, such that it is level with (e.g. forms part of the surface of) the at least one side of the sink. The UV light source may be any shape and size. The UV light source may be connected to a power source.

The first aperture, the second aperture and the UV light source are located below the countertop on the at least one side of the sink. That is, the first aperture, the second aperture and the UV light source are located inside the sink, below the plane of the substantially horizontal countertop. The may help to ensure that the water, the heated air and the UV light are provided directly into the interior volume of the sink.

When the sink comprises more than one side, the first aperture, the second aperture and the UV light source may each be located on any one of the sides of the sink. In some examples, each of the first aperture, the second aperture and the UV light source are located on different sides of the sink. In some examples, one or more of the first aperture, the second aperture and the UV light source may be located on the same side of the sink.

In some examples, the first aperture is configured to dispense water into the interior volume of the sink for washing a user's hands. This may allow a user to wash their hands inside the interior volume of the sink.

In some examples, the second aperture is configured to dispense heated air into the interior volume of the sink for drying a user's hands. This may allow a user to dry their hands inside the interior volume of the sink.

In some examples, the UV light source is configured to irradiate at least one side of the sink with UV light to disinfect the at least one side of the sink. The UV light source may be configured to disinfect at least one side of the sink owing to its wavelength, intensity and/or the direction in which the UV light is emitted.

In some examples, the sink unit comprises a sensor configured to detect the presence of a user's hands in the interior volume of the sink. In some examples, the sensor is an infrared sensor. The sensor may be located in any suitable and desired location on the sink unit. In some examples, the sensor is located below the countertop on the at least one side of the sink. Using a sensor to detect a person's hands in the interior volume of the sink may allow certain operations of the sink to be carried out automatically based on detecting the presence or absence of a user's hands inside the sink.

In some examples, the sink unit is configured to, when a person's hands are detected (e.g. by the sensor) in the interior volume of the sink:
automatically dispense water for a first predetermined period of time; and
when the first predetermined period of time has elapsed, automatically dispense heated air for a second predetermined period of time.

In some examples, the sink unit is configured to, when a person's hands are detected (e.g. by the sensor) in the interior volume of the sink:
automatically dispense water from the first aperture for a first predetermined period of time; and
when the first predetermined period of time has elapsed, automatically dispense heated air from the second aperture for a second predetermined period of time.

In some examples, the sink unit may be configured to dispense heated air immediately after the first period of time has elapsed (e.g. immediately after the sink unit has ceased dispensing water).

Therefore, the water and heated air for a person to wash and dry their hands may be provided in sequence when a person's hands are detected inside the sink, without any further input required from the user (e.g. turning on a tap, pressing a button, making any particular gesture).

The first predetermined period of time and the second predetermined period of time may be any suitable and desired time period. In some examples, the first predetermined period of time is between 10 seconds and 30 seconds, e.g. between 15 seconds and 25 seconds, e.g. approximately 20 seconds. In some examples, the second predetermined period of time is between 5 seconds and 15 seconds, e.g. approximately 10 seconds.

In some examples, the sink unit is configured to, when a person's hands are no longer detected (e.g. by the sensor) in the interior volume of the sink:
automatically irradiate at least one side of the sink with UV light from the UV light source for a third predetermined period of time.

In some examples, the sink unit is configured to, when a person's hands are no longer detected in the interior volume of the sink:
automatically irradiate at least one side of the sink with UV light for a third predetermined period of time.

Therefore, UV light for disinfecting at least one side of the sink may be provided after a person has finished washing and drying their hands and has removed their hands from the sink without any further input required from the user (e.g. pressing a button, making any particular gesture).

The third predetermined period of time may be any suitable and desired time period. In some examples, the third predetermined period of time is approximately 5 seconds.

In some examples, the sink unit comprises one or more lights configured to indicate whether the sink is dispensing water, dispensing heated air and/or emitting UV light. In some examples, there are three lights each corresponding to one of the functions of the sink unit (i.e. dispensing water, dispensing heated air and emitting UV light).

The light(s) may provide feedback to a user of the sink unit. Using lights to indicate which function the sink unit is performing may be advantageous when the sink unit is configured to operate automatically (e.g. on the basis of detecting a user's hands in the interior volume of the sink).

In some examples, the sink unit is configured for a user's hands to be placed vertically inside the interior volume of the sink when a user is washing their hands. The countertop may be positioned at approximately waist height when a person is standing. The sink may have a shape and size suitable for a person to place both of their hands into the interior volume of the sink. This may help to ensure that a person can wash their hands while their hands remain inside the interior volume of the sink, thereby helping to reduce the amount of water that splashes outside of the sink when a person washes their hands.

In some examples, the distance between the countertop and a bottom surface of the sink is at least 20 cm in a direction substantially perpendicular to the countertop, e.g. at least 22 cm, e.g. at least 24 cm, e.g. at least 26 cm. A distance of at least 20 cm between the countertop and a bottom surface of the sink may help to ensure that a user is able to place their hands vertically inside the interior volume of the sink.

In some examples, the first aperture, the second aperture and/or the UV light source extend in a strip substantially parallel to the countertop. That is, the first aperture, the second aperture and/or the UV light source have a length in a direction parallel to the countertop that is greater than their width in a direction perpendicular to the countertop. In some examples, the first aperture, the second aperture and/or the UV light source extend substantially parallel to the countertop over more than half of the width of the sink.

The shape and size of the first aperture and the second aperture helps to ensure that the water and heated air provided by the first and second apertures extend across the width of a user's hands (e.g. when held vertically in the interior volume of the sink), thereby helping to ensure that a user can wash their hands inside the interior volume of the sink. The shape and size of the UV light source helps to ensure that a greater proportion of the one or more surfaces of the sink are able to be irradiated with UV light.

In some examples, the sink unit comprises a third aperture configured to dispense water into the interior volume of the sink;
wherein the third aperture is located below the countertop on the at least one side of the sink; and
wherein optionally the third aperture has a smaller surface area than the first aperture.

The third aperture may be formed in the at least one side of the sink. The third aperture may be any shape and size. The third aperture may be in fluid communication with a source of water, e.g. the same source of water as for the first aperture. The third aperture may form an outlet for water from a water source.

The third aperture is located below the countertop on the at least one side of the sink. That is, third aperture is located inside the sink, below the plane of the substantially horizontal countertop. The may help to ensure that the water from the third aperture is provided directly into the interior volume of the sink.

When the sink comprises more than one side, the first aperture, the second aperture, the third aperture and the UV light source may each be located on any one of the sides of the sink. In some examples, each of the first aperture, the second aperture, the third aperture and the UV light source are located on different sides of the sink. In some examples, one or more of the first aperture, the second aperture, the third aperture and the UV light source may be located on the same side of the sink.

Owing to its smaller surface area, the third aperture may be configured to provide a more focused stream of water (e.g. a stream of water having a smaller cross sectional area) than the more extended source provided by the first aperture. Therefore, the first aperture and the third aperture may be better suited for different purposes. The first aperture may be adapted to provide a more extended water source for a user to wash their hands, whereas the third aperture may be adapted to provide a more focused stream of water for a user to perform other tasks, such as brushing their teeth or filling a water bottle or glass with water.

In some examples, the sink unit comprises one or more controls for controlling the temperature of the water dispensed from the third aperture. The controls may be any suitable and desired type, for example, one or more buttons, one or more switches and/or one or more knobs. In some examples, the sink unit comprises a first control corresponding to cold water being provided from the third aperture and a second control corresponding to warm water being provided from the third aperture. Providing control of the temperature of the water dispensed from the third aperture may be advantageous because the third aperture may be used for a variety of purposes that could require different temperature water.

In some examples, a first side of the sink comprises the first aperture and a first UV light source; and
a second opposing side of the sink comprises the second aperture and a second UV light source.

The first UV light source and the second UV light source may be similar or different (e.g. owing to their shape, size, relative locations, the wavelength of the UV light that they emit and/or the intensity of the UV light that they emit). Providing two UV light sources (in particular two UV light sources on opposing sides of the sink) may help to ensure that a greater proportion of the side(s) of the sink are irradiated with UV light.

Providing the first aperture and the second aperture on opposing sides of the sink may help to ensure that both apertures can be provided at a similar height relative to the countertop, which may be advantageous because the water and the heated air are both intended to be directed towards a user's hands.

In some examples, the first side of the sink is furthest from a user using the sink unit and the second opposing side of the sink is closest to a user using the sink unit.

In some examples, the sink unit comprises a third aperture configured to dispense water into the interior volume of the sink;
wherein a third side of the sink comprises the third aperture.

Together with its smaller surface area, the location of the third aperture compared to the location of the first aperture may help to ensure that they are each adapted for different purposes. The first aperture may be adapted to provide a more extended water source that extends across the width of a user's hands, making it more suitable for a user to wash their hands, whereas the third aperture may be adapted to provide a more focused stream of water in a different location and/or provided in a different direction for a user to perform other tasks, such as brushing their teeth or filling a water bottle or glass with water.

In some examples, the first side of the sink and the second side of the sink are substantially parallel to one another; and
the third side of the sink is substantially perpendicular to the first side of the sink and the second side of the sink.

The first side of the sink and the second side of the sink may each be connected (e.g. via a (rounded) corner) to the third side of the sink.

As the third aperture is located on a surface that is substantially perpendicular to that of the first aperture, the water from the third aperture may travel in a direction perpendicular to the direction of travel of the water from the first aperture. This may help to provide an alternative water source if the direction of travel of the water from the first aperture is not convenient for the task being performed. For example, this may help to provide a more ergonomic way for the user to perform tasks other that washing their hands in the sink (e.g. brushing their teeth or filling a water bottle or glass with water).

In some examples, the sink unit comprises:
a water tank configured to supply water to the first aperture; and
an air duct configured to supply heated air to the second aperture;
wherein optionally the sink unit comprises a third aperture configured to dispense water into the interior volume of the sink; and
wherein the water tank is configured to supply water to the third aperture.

The water tank may be in fluid communication with the first aperture and the third aperture, where present. The air duct may be in fluid communication with the second aperture.

The water tank may be configured to store water and supply cold water and/or warm water to the sink. In some examples, the water tank may be configured to heat the water before providing it to the first aperture and/or the third aperture.

In some examples, the water tank is approximately cuboid in shape, having a height, a width and a depth. In some examples, the depth of the water tank is smaller than the height and the width of the water tank.

When the water tank is mounted against a surface, this may help to ensure that the water tank extend outwards from the surface by a small amount owing to its small depth. For example, the water tank may be mounted to a wall of the aircraft lavatory unit. A water tank having a depth that is smaller than its height and its width may be considered to be more compact than a cylindrical water tank, for example. This may be advantageous because space is normally limited on board an aircraft, in particular in a lavatory unit of an aircraft.

In some examples, the air duct comprises:
an air inlet comprising a fan configured to draw air into the air duct;
a filter; and
a heater configured to heat the air in the duct.

The air inlet, the filter and the heater may be any suitable and desired type. The filter may be configured to remove objects such as dust and dirt from the air being drawn into the air duct. The filter may be configured to sanitise the air being drawn into the air duct. The filter may be configured to reduce unpleasant odours in the air being drawn into the air duct.

The heater may be configured to heat the air provided to the second aperture. In some examples, the heater comprises a heater coil wrapped around the air duct proximal to the sink.

### Brief Description of the Drawings

Certain examples of the present disclosure will now be described with reference to the accompanying drawings in which:
Figure 1 is a perspective view of a sink unit;
Figure 2 is a cross-sectional view of a sink unit;
Figure 3 is a plan view of a sink unit;
Figure 4 is a perspective view of a sink unit;
Figures 5a-d are perspective views of a user using a sink unit;
Figure 6 is a cross-sectional view of an aircraft lavatory unit comprising a sink unit; and
Figure 7 is a flow chart showing a method of operating a sink unit.

### Detailed Description

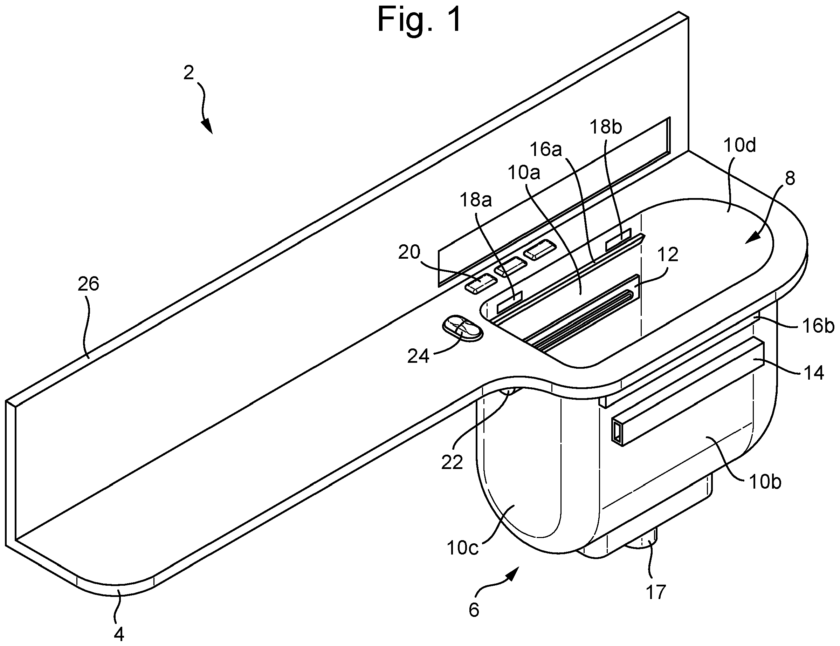

Figure 1 is a perspective view of a sink unit 2.

The sink unit 2 includes a substantially horizontal countertop 4 and a sink 6 extending downwards from the countertop 4. In the sink unit 2 of Figure 1, the countertop 4 extends around the sink 6 and outwards to one side of the sink 6. However, it will be understood that the countertop 4 may be any type.

The sink 6 includes an interior volume 8 defined by the sides 10a-d of the sink 6. In the sink unit 2 of Figure 1, the sink 6 comprises four sides 10a-d. The first side 10a of the sink 6 and the second side 10b of the sink are substantially parallel and opposite one another. The first side 10a of the sink 6 is furthest from a user using the sink unit 2 and the second (opposing) side 10b of the sink 6 is closest to a user using the sink unit 2. The third side 10c of the sink is connected to the first side 10a and the second side 10b and is substantially perpendicular to both. The fourth side 10d is opposite the third side 10c, is connected to the first side 10a and the second side 10b and has a semi-circular cross-section.

The four sides 10a-d of the sink 6 are connected to one another to form the bottom of the sink 6. The volume of the sink 6 may be defined by the sides of the sink, the bottom of the sink 6 and an (imaginary) surface in the plane of the countertop 4.

The sink 6 includes a first aperture 12 configured to dispense water into the interior volume 8 of the sink 6 and a second aperture 14 configured to dispense heated air into the interior volume 8 of the sink 6. The first and second apertures 12, 14 in the sink 6 are configured to provide the water and heated air required for a user to wash and dry their hands in the sink 6.

The sink 6 includes two UV light sources 16a, 16b configured to irradiate the interior volume 8 of the sink 6 with UV light. The UV light sources are configured to irradiate the sides 10a-d of the sink 6 with UV light to disinfect the sides 10a-d of the sink 6 (e.g. owing to the wavelength and/or the intensity if the UV light).

The first aperture 12, the second aperture 14 and the UV light sources 16a, 16b are located below the countertop 4 on the sides 10a-d of the sink 6. Hence, the sink 6 may be an appropriate shape and size for a user's hands to be inserted vertically into the interior volume 8 of the sink 6 (e.g. in order to access the water and heated air inside the interior volume 8 of the sink 6). This helps to ensure that the water, heated air and UV light are directed into the interior volume 8 of the sink 6, thereby helping to keep the lavatory unit of the aircraft clean and dry.

In the sink unit 2 of Figure 1, the first side 10a of the sink comprises the first aperture 12 and the first UV light source 16a. The second (opposing) side 10b of the sink comprises the second aperture 14 and the second UV light source 16b. Providing two UV light sources 16a, 16b on opposing sides 10a, 10b of the sink 6 of Figure 1 helps to ensure that all of the sides 10a-d of the sink 6 may be irradiated with UV light. Providing the first aperture 12 and the second aperture 14 on opposing sides 10a, 10b of the sink 6 helps to ensure that both apertures 12, 14 can be provided at the same height relative to the countertop 4, which is advantageous because the water and heated air are both intended to be directed towards a user's hands.

In the sink unit 2 of Figure 1, the first aperture 12, the second aperture 14 and each of the UV light sources 16a, 16b extend over the majority of the width of the sink 6 in a strip substantially parallel to the countertop 4. This helps to ensure that the water and heated air provided by the first and second apertures 12, 14 respectively are configured to provide water and hot air across the width of a user's hands (held vertically in the interior volume 8 of the sink 6).

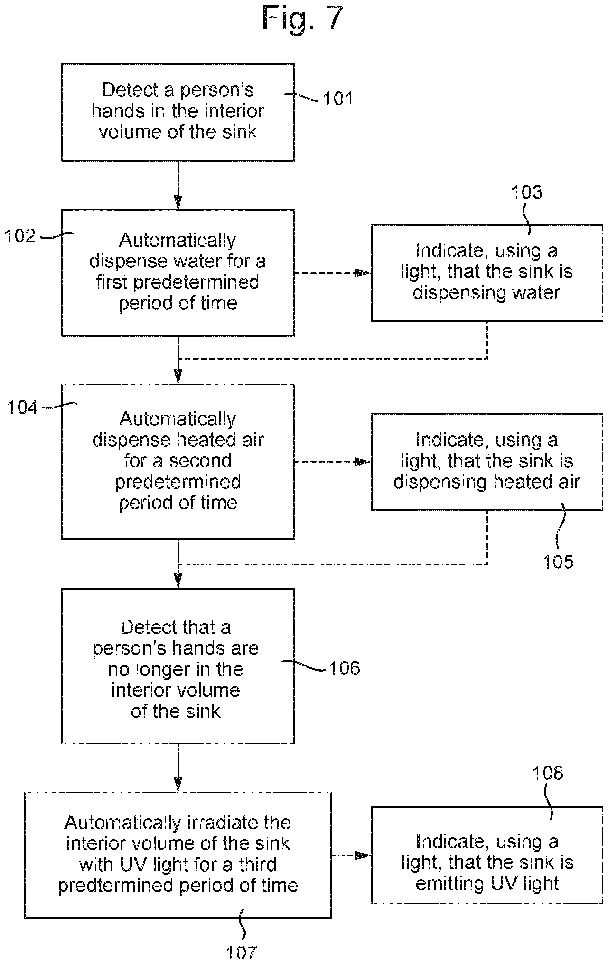

The sink unit 2 of Figure 1 includes two sensors 18a, 18b configured to detect the presence of a user's hands in the interior volume 8 of the sink 6. The sensors 18a, 18b may be infrared sensors. Upon detecting a person's hands in the interior volume 8 of the sink 6, the sink unit may be configured to automatically dispense water, automatically dispense heated air and automatically irradiate the sink 6 with UV light. This will be discussed in more detail in relation to Figure 7.

As shown in Figure 1, the sink unit 2 may include lights 20 configured to indicate (to a user) whether the sink is dispensing water, dispensing heated air or emitting UV light. There may be three lights 20, which may each correspond to one of the functions of the sink unit 2 (i.e. dispensing water, dispensing heated air or emitting UV light). This may be particularly advantageous when the sink unit 2 is configured to operate automatically on the basis of detecting a user's hands in the interior volume 8 of the sink 6.

As shown in Figure 1, the sink unit 2 may include a third aperture 22 configured to dispense water into the interior volume 8 of the sink 6. The third aperture 22 may be located below the countertop 4 on the third side 10c of the sink 6. The third aperture 22 may have a smaller surface area than the first aperture 12. Owing to its shape, the third aperture 22 may be configured to provide a more focused stream of water than the extended source provided by the first aperture 12. Therefore, the first aperture 12 and the third aperture 22 may be adapted for different purposes. The first aperture 12 may be adapted to provide an extended water source for a user to wash their hands, whereas the third aperture 22 may be adapted to provide a more focused stream of water for a user to perform other tasks, such as brushing their teeth or filling a water bottle or glass with water.

As shown in Figure 1, the third aperture 22 may be located on the third side 10c of the sink 6. Hence, the water from the third aperture 22 may travel in a direction perpendicular to the user (and perpendicular to the direction of travel of the water from the first aperture 12). This may help to provide a more ergonomic way for the user to perform tasks other that washing their hands in the sink 6 (e.g. brushing their teeth or filling a water bottle or glass with water).

As shown in Figure 1, the sink unit 2 may include buttons 24 for controlling the temperature of the water dispensed from the third aperture 22. The two buttons 24 may correspond to cold and warm water respectively. Providing control of the temperature of the water dispensed from the third aperture 22 may be advantageous because the third aperture 22 may be used for a variety of purposes that could require different temperature water.

As shown in Figure 1, the sink unit 2 may include a drain 17 located at the bottom of the sink 6, at a point furthest from the countertop 4.

As shown in Figure 1, the sink unit 2 may include a substantially vertical surface 26 connected to the countertop 4. This surface 26 may be configured as a splashback, protecting an interior wall of the aircraft from any splashes of water from the sink 6.

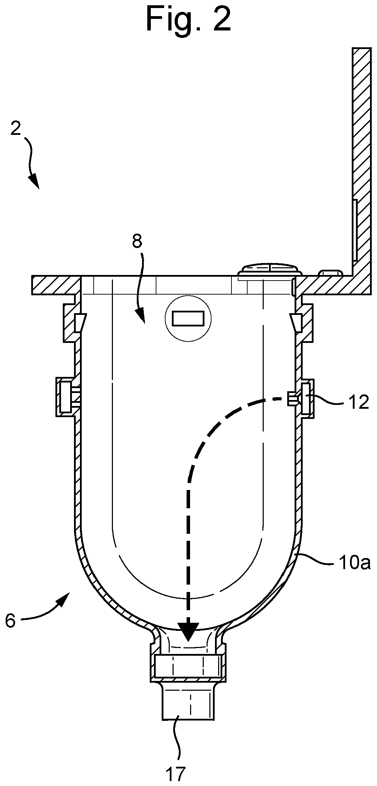

Figure 2 is a cross-sectional view of a sink unit 2. As shown in Figure 2, the first aperture 12 may be configured to provide water that travels outwards from the first surface 10a into the interior volume 8 of the sink 6. In this example, the water travels in a curved (e.g. parabolic) path towards the drain 17 at the bottom of the sink 6. It can be seen that a user may place their hands into the path of the water in order to wash their hands.

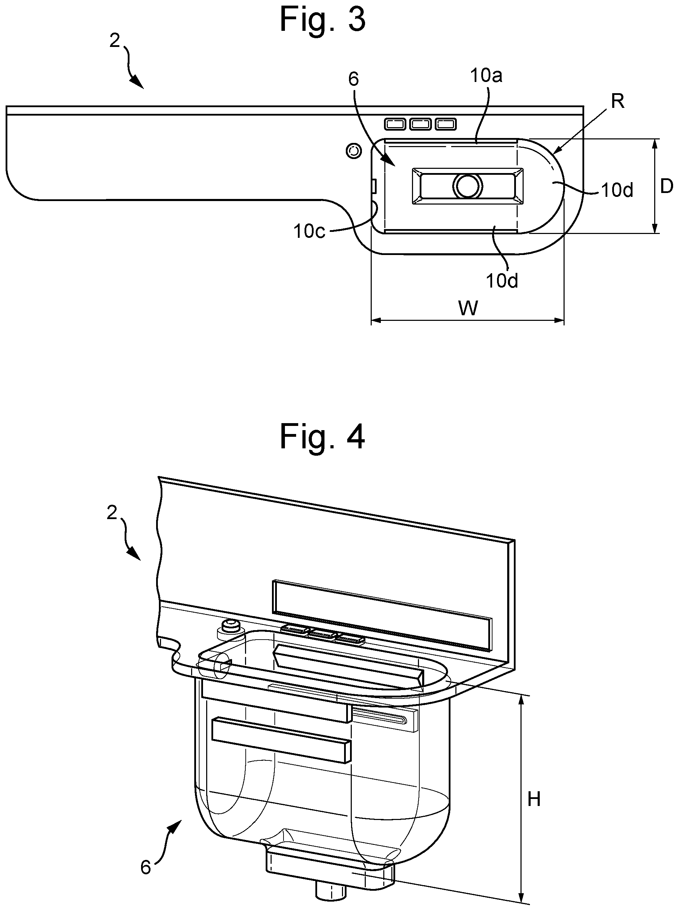

Figure 3 is a plan view of a sink unit 2. Some example dimensions of the sink 6 are indicated on Figure 3. The depth D of the sink 6 is measured as the distance between the first surface 10a and the second surface 10b, in a direction perpendicular to the first surface 10a and the second surface 10b. In some examples, the depth D of the sink 6 may be at least 10 cm, e.g. at least 12 cm, e.g. at least 14 cm, e.g. at least 16 cm.

The width W of the sink 6 is measured as the distance between the third surface 10c and the outermost edge of the fourth surface 10d, in a direction perpendicular to the third surface 10c and the outermost edge of the fourth surface 10d. In some examples, the width W of the sink 6 may be at least 26 cm, e.g. at least 28 cm, e.g. at least 30 cm, e.g. at least 32 cm.

The radius of curvature R of the fourth surface 10d is indicated on Figure 3. In this example, the radius of curvature R is half of the depth D of the sink 6. In some examples, the radius of curvature R of the fourth surface 10d may be at least 5 cm, e.g. at least 6 cm, e.g. at least 7 cm, e.g. at least 8 cm.

Figure 4 is a perspective view of a sink unit 2. The height H of the sink 6 is indicated on Figure 4. The height H of the sink 6 is measured as the distance between the bottom surface of the sink 6 and the plane of the countertop 4, in a direction perpendicular to the countertop 4. In some examples, the height H of the sink 6 may be at least 20 cm, e.g. at least 22 cm, e.g. at least 24 cm, e.g. at least 26 cm.

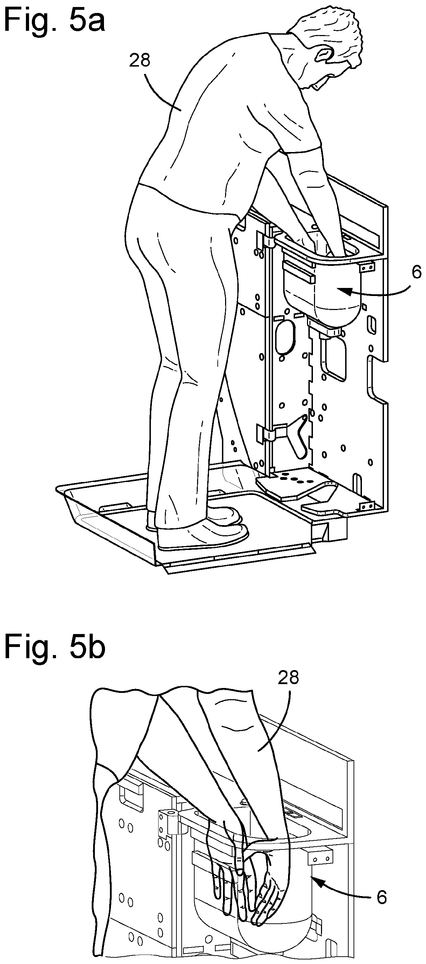

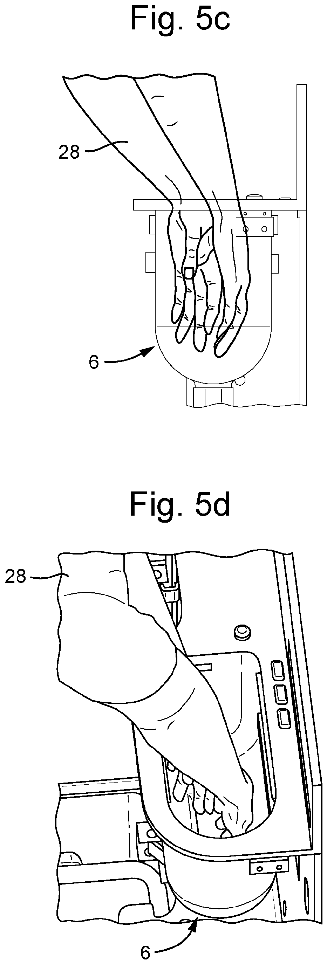

Hence, the dimensions of the sink 6 may be such that a user can place their hands vertically into the interior volume 8 of the sink. This can be seen in Figures 5a-d, which are perspective views of a user 28 using a sink unit 2. The user 28 of Figures 5a-d is a male having a height in the 95^{th} percentile. Therefore, it can be seen that the sink 6 may be a suitable size for most people to use the sink 6 for washing their hands by inserting their hands vertically into the interior volume 8 of the sink 6.

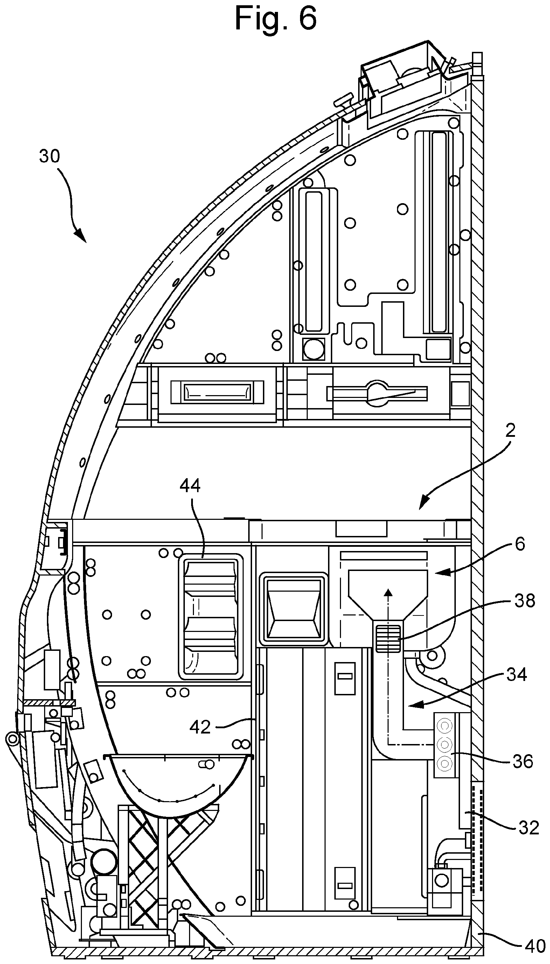

Figure 6 is a cross-sectional view of an aircraft lavatory unit 30 comprising a sink unit 2.

The sink unit 2 of Figure 6 includes a water tank 32 and an air duct 34. The water tank 32 is configured to supply water to the first aperture of the sink 6 and the third aperture of the sink 6, where present. The water tank 32 may be configured to supply cold water and/or warm water to the sink 6.

In the sink unit 2 of Figure 6, the water tank 32 is approximately cuboid in shape. The water tank 32 has a height (extending vertically), a width (extending backwards into the page) and a depth (extending from left to right). The depth of the water tank 32 may be smaller than the height and the width of the water tank 32. In this example, this configuration allows the water tank 32 to be mounted against a wall 40 of the aircraft lavatory unit 30 while minimising the extent to which the water tank 32 extends outwards from the wall 40. Therefore, this water tank 32 may be considered to be more compact than a cylindrical water tank, for example.

The air duct 34 is configured to supply heated air to the second aperture of the sink 6. The air duct 34 may include an air inlet 36 including a fan configured to draw air into the air duct 34, a filter (not shown) and a heater 38 configured to heat the air in the duct. The air inlet 36, the filter and the heater 38 may be any suitable and desired type. The filter may be configured to remove objects such as dust and dirt from the air being drawn into the air duct. The filter may be configured to sanitise the air being drawn into the air duct. The filter may be configured to reduce unpleasant odours in the air being drawn into the air duct. In this example, the heater 38 may comprise a heater coil wrapped around the air duct 34 proximal to the sink 6.

As shown in Figure 6, the aircraft lavatory unit 30 may include a waste bin 42 and a tissue paper holder 44.

Figure 7 is a flow chart showing an example method of operating a sink unit 2.

The method begins at step 101, whereby a person's hands are detected in the interior volume of the sink. When a person's hands are detected in the interior volume of the sink, the method proceeds to step 102, whereby the sink unit automatically dispenses water for a first predetermined period of time. Optionally, the method proceeds to step 103, whereby it is indicated, using a light, that the sink is dispensing water. After step 102 (and optionally step 103), the method proceeds to step 104, whereby the sink unit automatically dispenses heated air for a second predetermined period of time. Optionally, the method proceeds to step 105, whereby it is indicated, using a light, that the sink is dispensing heated air.

After step 104 (and optionally step 105), the method proceeds to step 106, whereby it is detected that a person's hands are no longer in the interior volume of the sink. The method proceeds to step 107, whereby the sink unit automatically irradiates the interior volume of the sink with UV light for a third predetermined period of time. Optionally, the method proceeds to step 108, whereby it is indicated, using a light, that the sink is emitting UV light.

In some examples, the first predetermined period of time is 20 seconds, the second predetermined period of time is 10 seconds and the third predetermined period of time is 5 seconds.

In some examples, the sink unit may be configured to proceed from step 102 (and optionally step 103) to step 104 (and optionally step 105) immediately after the first period of time has elapsed (e.g. immediately after the sink unit has ceased dispensing water). In some examples, the sink unit may be configured to proceed from step 106 to step 107 (and optionally step 108) immediately after the person's hands are no longer detected in the interior volume of the sink.

Providing a sink unit with an outlet for water and heated air inside the sink, below the level of the countertop, helps to ensure that the water and heated air are directed into the interior volume of the sink when a user washes their hands. This helps to reduce the amount of water that spills out of the sink onto other surfaces in the lavatory unit, thereby helping to keep the lavatory unit of the aircraft clean and dry.

In order to facilitate the user accessing the water and heated air inside the sink, the sink may have a shape and size that facilitates a user being able to insert their hands vertically inside the sink. Furthermore, the water and heated air may each be provided by an extended source that provides water/heated air across the width of a person's hands. Therefore, a user may wash their hands in the vertical position.

Providing a UV light inside the sink, below the level of the countertop, helps to ensure that the sink may be sanitised between uses. This is particularly important on busy aircraft where the sink may be used many times during a flight.

By providing the water and heated air automatically for a predetermined amount of time, an appropriate amount of water and heated air may be provided for a person to wash and dry their hands. This may help to prevent wasted energy being used in heating water or air that is not required. Furthermore, automating the provision of water and heated air may reduce the number of surfaces that a user needs to touch. This may be considered more hygienic.

Providing UV light automatically after a person's hands have been removed from the sink may help to ensure that the UV light is being emitted safely. Furthermore, this may help to ensure that a person's hand is not physically blocking the UV light from reaching one or more surfaces of the sink.

Finally, providing a heated air outlet inside the sink, in particular one which is automated, may encourage a person to use fewer paper towels, which may help to decrease the amount of waste produced on board the aircraft. This is particularly advantageous on board an aircraft where space is limited and the time to clean the aircraft in between flights may be limited.

## Claims

1. A sink unit for an aircraft, wherein the sink unit comprises:
a substantially horizontal countertop; and
a sink extending downwards from the countertop;
wherein the sink comprises an interior volume defined by at least one side of the sink; and
wherein the sink comprises:
a first aperture configured to dispense water into the interior volume of the sink;
a second aperture configured to dispense heated air into the interior volume of the sink; and
a UV light source configured to irradiate the interior volume of the sink with UV light;
wherein the first aperture, the second aperture and the UV light source are located below the countertop on the at least one side of the sink.

2. The sink unit as claimed in claim 1, wherein the first aperture is configured to dispense water into the interior volume of the sink for washing a user's hands.

3. The sink unit as claimed in claim 1 or 2, wherein the second aperture is configured to dispense heated air into the interior volume of the sink for drying a user's hands.

4. The sink unit as claimed in claim 1, 2 or 3, wherein the UV light source configured to irradiate at least one side of the sink with UV light to disinfect the at least one side of the sink.

5. The sink unit as claimed in any one of the preceding claims, wherein the sink unit comprises a sensor configured to detect the presence of a user's hands in the interior volume of the sink.

6. The sink unit as claimed in claim 5, wherein the sink unit is configured to, when a person's hands are detected in the interior volume of the sink:
automatically dispense water for a first predetermined period of time; and
when the first predetermined period of time has elapsed, automatically dispense heated air for a second predetermined period of time.

7. The sink unit as claimed in claim 6, wherein the sink unit is configured to, when a person's hands are no longer detected in the interior volume of the sink:
automatically irradiate at least one side of the sink with UV light for a third predetermined period of time.

8. The sink unit as claimed in any one of the preceding claims, wherein the sink unit comprises one or more lights configured to indicate whether the sink is dispensing water, dispensing heated air and/or emitting UV light.

9. The sink unit as claimed in any one of the preceding claims, wherein the sink unit is configured for a user's hands to be placed vertically inside the interior volume of the sink when a user is washing their hands.

10. The sink unit as claimed in any one of the preceding claims, wherein the first aperture, the second aperture and/or the UV light source extend in a strip substantially parallel to the countertop.

11. The sink unit as claimed in any one of the preceding claims, wherein the sink unit comprises a third aperture configured to dispense water into the interior volume of the sink;
wherein the third aperture is located below the countertop on the at least one side of the sink; and
wherein optionally the third aperture has a smaller surface area than the first aperture.

12. The sink unit as claimed in claim 11, wherein the sink unit comprises one or more controls for controlling the temperature of the water dispensed from the third aperture.

13. The sink unit as claimed in any one of the preceding claims, wherein a first side of the sink comprises the first aperture and a first UV light source; and
a second opposing side of the sink comprises the second aperture and a second UV light source.

14. The sink unit as claimed in claim 13, wherein the sink unit comprises a third aperture configured to dispense water into the interior volume of the sink;
wherein a third side of the sink comprises the third aperture.

15. The sink unit as claimed in any one of the preceding claims, wherein the sink unit comprises:
a water tank configured to supply water to the first aperture; and
an air duct configured to supply heated air to the second aperture;
wherein optionally the sink unit comprises a third aperture configured to dispense water into the interior volume of the sink; and
wherein the water tank is configured to supply water to the third aperture.
